Europäisches Patentamt

(19) European Patent Office

Office européen des brevets

(11) **EP 0 486 351 B1**

(12) **FASCICULE DE BREVET EUROPEEN**

(45) Date de publication et mention
de la délivrance du brevet:
**09.07.1997 Bulletin 1997/28**

(51) Int. Cl.⁶: **C07C 55/07**, C07C 51/41, C01F 17/00

(21) Numéro de dépôt: **91402924.4**

(22) Date de dépôt: **31.10.1991**

(54) **Procédé de fabrication d'oxalates doubles de terres rares et d'ammonium et leurs utilisations pour la fabrication d'oxydes de terres rares**

Verfahren zur Herstellung von gemischten Oxalaten von Ammonium und seltenen Erden und deren Anwendung zur Herstellung von Oxyden der seltenen Erden

Method for the production of mixed ammonium-rare earth oxalates and their application to the production of rare earth oxides

(84) Etats contractants désignés:
**AT BE CH DE DK ES FR GB GR IT LI LU NL SE**

(30) Priorité: **13.11.1990 FR 9014031**

(43) Date de publication de la demande:
**20.05.1992 Bulletin 1992/21**

(73) Titulaire: **RHONE-POULENC CHIMIE
92408 Courbevoie Cédex (FR)**

(72) Inventeur: **David, Claire
F-75008 Paris (FR)**

(74) Mandataire: **Dutruc-Rosset, Marie-Claude et al
RHONE-POULENC CHIMIE,
Direction de la Propriété Industrielle,
25, Quai Paul Doumer
92408 Courbevoie Cédex (FR)**

(56) Documents cités:
**DE-A- 2 915 396**

- **PATENT ABSTRACTS OF JAPAN vol. 10, no. 4 (C-322)(2061) 09 janvier 1986, & JP-A-60 166222 (MITSUBISHI KINZOKU) 29 août 1985,**
- **"Gmelin Handbook of Inorganic Chemistry System No. 39, Part D5" 1984, Springer Verlag, Berlin RFA**

## Description

La présente invention concerne un procédé de fabrication d'oxalates doubles de terres rares et d'ammonium, leur utilisation pour l'obtention d'oxyde de terres rares .

Elle se rapporte plus particulièrement à un procédé permettant d'obtenir des oxalates doubles présentant une morphologie et une granulométrie déterminées.

Les oxydes de terres rares trouvent de nombreuses applications dans le domaine notamment de la céramique et de l'électronique mais à l'heure actuelle, on constate sur le marché une demande croissante de produits à granulométrie contrôlée.

Une des voies classiques pour obtenir des oxydes de terres rares et qui est largement décrite dans la littérature, notamment dans le NOUVEAU TRAITE DE CHIMIE MINERALE, Tome VII, (1959), P. 1007 de Paul PASCAL, consiste à calciner entre 500 et 900°C les oxalates de terres rares obtenus par précipitation à l'aide de l'acide oxalique, des sels de terres rares sous forme de solution aqueuse. Cependant, un tel procédé de fabrication ne conduit qu'à des oxydes de terres rares présentant une grosse granulométrie.

Il est également connu selon JP 53 095911-A (Chemical Abstracts 90, 40940 w) de préparer des oxydes de terres rares fins et plus particulièrement de l'oxyde d'yttrium fin par calcination d'un oxalate d'yttrium et d'ammonium qui consiste à partir d'une solution aqueuse d'un sel d'yttrium, à précipiter l'yttrium sous la forme de son hydroxyde préparé par réaction de la solution aqueuse d'un sel d'yttrium et d'une solution aqueuse basique comme l'ammoniaque puis à traiter la bouillie d'hydroxyde résultante par l'acide oxalique, et enfin à séparer le précipité obtenu, à le laver et à le calciner à une température de 750°C. Ledit procédé conduit conformément à la description donnée dans JP 53-095911-A à l'obtention d' oxyde d'yttrium fin. Le diamètre des particules est compris entre 0,9 et 4,5 μm, les cristaux ayant une forme de plaquettes à bords arrondis.

Toutefois, le contrôle de la dimension des particules ainsi que de la répartition granulométrique est relativement difficile car les conditions de mise en oeuvre du procédé influent fortement sur celles-ci.

Les deux articles du Bull. Chem. Soc. Japan 1990 63 , 2115 et 63 ,378 décrivent des méthodes de préparation de poudres d'oxyde d'yttrium par calcination de précipités qui sont obtenus respectivement par réaction de nitrate d'yttrium et d'ammoniaque, puis addition d'acide oxalique et par réaction d'ammoniaque avec une solution d'oxalate d'yttrium. Dans ces deux cas, on obtient des produits de granulométrie peu ressérée et de taille moyenne d'au plus 1 μm.

L'article du J. Inorg. Nucl. Chem. 1964, vol. 26 , pp. 931-936 décrit des méthodes de précipitation analogues par ajout d'oxalate d'ammonium à une solution de nitrate d'yttrium ou d'ammoniaque à des solutions acides de nitrate.

Le Gmelin Handbook of Inorganic Chemistry (1984),part D5,System n° 39, pp. 141, 145 fait état d'oxalates doubles préparés par addition d'acide oxalique, puis de $NH_3$ à des solutions de nitrates de terres rares.

Un des buts de la présente invention est notamment de remédier à ces inconvénients en proposant un procédé de fabrication d'oxalates doubles d' ammonium et de terres rares présentant une distribution granulométrique resserrée, avec des dimensions moyennes des cristaux qui peuvent être contrôlées .

Par oxalate double de terres rares et d'ammonium, il faut comprendre un composé comprenant une ou plusieurs terres rares associées avec des ions ammonium et oxalate permettant après calcination de produire des oxydes simples ou mixtes.

Par l'expression terres rares il faut comprendre les élements portant les numéros atomiques compris entre 57 et 71 (bornes incluses) appartenant à la famille des Lanthanides, ainsi que l'yttrium portant le numéro atomique 39 .

A cet effet, l'invention propose un procédé de fabrication d'un oxalate double de terres rares et d'ammonium caractérisé en ce qu'il consiste :

- a mélanger, en milieu aqueux, au moins un composé capable de libérer des ions oxalates en solution avec au moins un composé de terres rares soluble dans l'eau et, un sel d'ammonium,
- a séparer le précipité obtenu
- et, éventuellement le sécher.

Selon une caractéristique de l'invention, le sel d'ammonium est choisi parmi le nitrate d'ammonium, le chlorure d'ammonium, l'acétate d'ammonium.

Ainsi, l'utilisation d'un sel d'ammonium, code par exemple le nitrate d'ammonium qui est le composé préféré de l'invention, permet un meilleur contrôle de la précipitation de l'oxalate double car il précipite directement sans précipitation au préalable de l'hydroxyde de terres rares comme dans le procédé antérieur cité cidessus . Cette précipitation directe permet de contrôler la granulométrie et la dispersion des tailles de cristallites de l'oxalate double précipité .

Selon un premier mode de réalisation préférée de l'invention, le sel d'ammonium et le composé de terres rares sont mélangés en milieu aqueux pour former une première solution .

Dans ce mode de réalisation, le composé capable de libérer en solution des ions oxalates en solution est ajouté à la première solution.

L'état ou la concentration de ce composé permet de modifier la taille des cristallites d'oxalate double précipité ainsi que leur répartition granulométrique .

Avantageusement, le composé capable de libérer des ions oxalates est choisi parmi l'acide oxalique cristallisé ou en solution, les oxalates d'alcalins cristallisés ou en solution, ou analogues.

Ainsi, l'addition du composé capable de libérer des ions oxalates sous forme solide permet d'obtenir un précipité avec des cristallites de faible taille, par exemple de l'ordre de 1 à 4 $\mu$m, tandis que l'addition de ce composé sous la forme d'une solution aqueuse, formant ainsi une seconde solution, permet d'obtenir des tailles de cristallites plus élevées.

Dans un second mode de réalisation de l'invention, la première solution contenant un sel d'ammonium et un composé de terres rares est ajoutée dans la seconde solution contenant le composé capable de libérer des ions oxalates.

Toutefois dans ce mode de réalisation le composé de terres rares et le sel d'ammonium peuvent être ajoutés sous forme de solution ou sous forme cristallisée. Ces composés peuvent être ajoutés soit sous forme de mélange solide ou de solution commune soit sous forme séparée mais alors ils doivent être de préférence ajoutés simultanémment dans la seconde solution.

Comme composés de terres rares convenables pour l'invention, on peut citer des nitrates, chlorures, sulfates, par exemple, ou un mélange de sels de terres rares, les nitrates étant les composés préférés de l'invention.

Parmis ces composés, le nitrate d'yttrium, nitrate d'europium, nitrate de lanthane, nitrate de néodyme, nitrate de dysprosium, nitrate de cérium, nitrate de gadolinium, nitrate de terbium ou un mélange de ceux-ci sont préférés.

En particulier, on peut mettre en oeuvre une solution aqueuse contenant des sels de terres rares qui proviendrait directement ou indirectement du traitement des minerais de terres rares.

Bien que le procédé de l'invention s'applique tout-à-fait bien aux terres rares cériques, il convient plus particulièrement aux terres rares yttriques.

On entend par "terres rares cériques", les éléments les plus légers des terres rares commençant avec le lanthane et s'étendant jusqu'au néodyme conformément au numéro atomique, et l'on désigne par "terres rares yttriques" les éléments les plus lourds des terres rares conformément au numéro atomique, commençant avec le samarium et finissant avec le lutécium et comprenant l'yttrium.

La concentration en composé de terres rares n'est pas critique.

Selon une autre caractéristique préférée du procédé, les rapports molaires $C_2O_4^=$/TR et $NH_4^+$/TR à la fin de la précipitation sont supérieurs ou égaux à 2, de préférence supérieurs ou égaux à 2,5.

Toutefois, la concentration en ions $(C_2O_4)^=$ et $NH_4^+$ dans les solutions n'est pas critique et peut varier dans de larges limites.

Selon un mode de réalisation préféré, on déterminera les concentrations en oxalate ainsi que la concentration en terre rare et ions ammonium dans les différentes solutions et les volumes des première et seconde solutions ainsi que les masses de produits cristallisés ajoutés pour obtenir dans le milieu réactionel final un rapport molaire entre l'ion oxalate et les terres rares $((C_2O_4)^=$/TR) supérieur ou égal à 2, avantageusement supérieur ou égal à 2,5 et un rapport ammonium à terres rares ($NH_4^+$/TR) supérieur ou égal à 2, de préférence supérieur ou égal à 2,5.

Les conditions de mise en oeuvre du procédé sont peu critiques pour obtenir un oxalate double. Toutefois, le contrôle de la vitesse de mélange des différentes solutions ou la vitesse d'introduction des produits cristallisés dans les solutions, de la température, de l'agitation du mélange permet de modifier et contrôler la morphologie de l'oxalate double précipité.

Par ailleurs, la température a une influence sur le rendement de la précipitation car le coefficient de solubilité de l'oxalate double augmente avec l'élévation de la température.

Selon une caractéristique préférentielle de l'invention, la précipitation est réalisée à une température comprise entre 30°C et 90°C, de préférence entre 35°C et 70°C.

Selon une autre caractéristique préférentielle de l'invention, la séparation du précipité est réalisée entre 5 min et 2 H environ après la fin de précipitation. Pendant cette période, le milieu réactionel peut être maintenu agité ou non.

Cette étape permet un réarrangement des cristaux et est généralement appelée une étape de mûrissement du précipité.

Le précipité obtenu est séparé du liquide surnageant par tout procédé de séparation solide/liquide comme par exemple, filtration, centrifugation décantation ou analogue. Il peut également être soumis à un ou plusieurs lavages, pour, par exemple, éliminer les sels solubles.

L'oxalate double de terres rares et d'ammonium peut subir un séchage pour évaporer l'eau non lié par exemple par un traitement thermique entre 50°C et 100°C ou un séchage sous pression réduite.

Le procédé de l'invention permet de produire un oxalate double de terres rares et d'ammonium présentant une granulométrie homogène, ainsi qu'une répartition granulométrique des cristaux très resserrée.

Les cristaux obtenus ont, par exemple, une taille comprise entre 1 et 4 $\mu$m environ et une forme de plaquette.

Une des utilisations de ces oxalates doubles de terres rares et d'ammonium est la production d'oxyde de terres rares obtenues par décomposition thermique de ceux-ci.

La morphologie et granulométrie des oxydes de terres rares obtenus par décomposition d'un oxalate double est généralement semblable à celle dudit oxalate double utilisé comme précurseur. Toutefois, selon les conditions de traitement thermique de l'oxalate double, la granulométrie de l'oxyde peut être légèrement différente de celle de l'oxalate.

Le traitement thermique ou calcination est généralement réalisé à une température comprise entre 600°C et 1200°C, avantageusement entre 800°C et 1000°C.

La durée de calcination est déterminée de manière classique par le contrôle du poids constant. A titre indicatif, la durée de la calcination peut varier entre 30 minutes et 6 heures environ.

L'invention sera illustrée par des exemples donnés ci-dessous uniquement à titre indicatif.

Exemple 1

Une solution contenant du nitrate d'yttrium à 1,36 mole/l est mélangée avec une solution de nitrate d'ammonium 2 M pour obtenir un rapport $NH_4^+/Y = 5$.

Cette solution est chauffée à 45°C.

85g d'acide oxalique cristallisé sont ajoutés pour ainsi avoir un rapport $(C_2O_4)^=/Y = 2,5$.

Le mélange est maintenu sous agitation pendant une heure à cette température.

Le précipité est ensuite récupéré par filtration et lavé à l'eau.

Le précipité lavé est séché à 100°C puis analysé par rayon X pour confirmer sa structure d'oxalate double d'yttrium et d'ammonium.

Ce sel est transformé en oxyde par calcination à 900°C pendant 1 heure.

L'analyse granulométrique réalisée au moyen du granulomètre CILAS ® montre que l'oxyde présente un diamètre moyen de particules de 2,55 µm, avec un facteur $\frac{\sigma}{m}$ de dispersibilité granulométrique de 0,53

le facteur $\frac{\sigma}{m}$ est calculé par la formule

$$\frac{\sigma}{m} = \frac{\varnothing_{84} - \varnothing_{16}}{2\,\varnothing_{50}}$$

dans laquelle :

$\varnothing_{84}$ : représente le diamètre pour lequel 84 % des particules ont un diamètre inférieur ou égal à $\varnothing_{84}$.

$\varnothing_{16}$ : représente le diamètre pour lequel 16 % de particules ont un diamètre inférieur ou égal à $\varnothing_{16}$.

$\varnothing_{50}$ : représente le diamètre moyen des particules.

Exemple 2

De manière analogue à l'exemple 1, on réalise une solution contenant du nitrate d'yttrium et du nitrate d'ammonium avec un rapport $NH_4^+/Y = 3,90$.

On ajoute dans cette solution, après l'avoir portée à 45°C, une solution d'acide oxalique 1 M de manière à obtenir un rapport $C_2O_4^=/Y = 2$.

Le milieu réactionnel est ensuite maintenu sous agitation pendant 30 minutes.

Après filtration et lavage à l'eau, le précipité est séché à 100°C puis calciné à 900°C pour le transformer en oxyde d'yttrium.

L'analyse granulométrique de cet oxyde montre qu'il a un diamètre moyen $\varnothing_{50}$ égal à 13,6 µm et un $\frac{\sigma}{m}$ égal à 0,51.

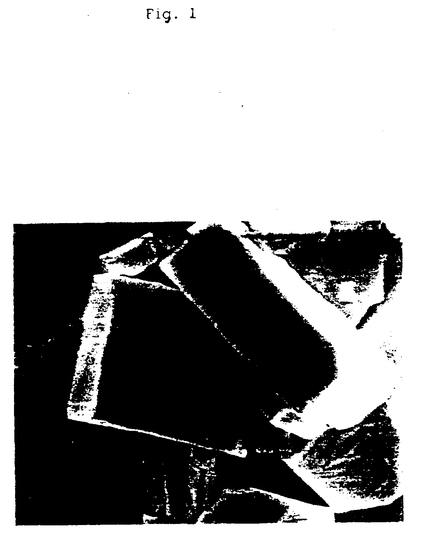

Les cristaux de cet oxyde sont illustrés à la figure unique (grossissement 6000 fois).

Exemple 3

Une solution contenant 0,675 mole d'acide oxalique cristallisé est chauffée à 45°C.

On ajoute à cette solution, une seconde solution contenant un nitrate d'ammonium et un nitrate d'yttrium pour obtenir dans le milieu réactionnel les rapports suivants.

$$C_2O_4^=/Y = 2,5$$

$$NH_4^+/Y = 4,96$$

Le mélange est maintenu sous agitation pendant 1 heure puis le précipité est filtré et lavé à l'eau.

Après séchage à 100°C, l'oxalate double est calciné pendant 1 heure à 900°C.

L'analyse granulométrique montre qu'il a un $\varnothing_{50}$ égal à 4,06 µm et un $\frac{\sigma}{m} = 0,48$.

Exemple 4

Dans une solution d'acide oxalique chauffée à 60°C on ajoute simultanément une solution de nitrate d'yttrium et une solution de nitrate d'ammonium, de manière à obtenir dans le milieu réactionnel les rapports suivants :

$$C_2O_4^=/Y = 2,5$$

$$NH_4^+/Y = 5,15$$

Le mélange est maintenu sous agitation pendant une heure.

Le précipité est ensuite filtré et lavé à l'eau, puis séché et calciné comme dans les exemples précédents.

L'oxyde ainsi obtenu présente un diamètre moyen de particules égal à 3 µm et un $\frac{\sigma}{m}$ égal à 0,6.

Exemple 5

L'exemple 1 est reproduit mais en utilisant une solution de chlorure d'yttrium et chlorure d'ammonium à la place de la solution de nitrate d'yttrium et nitrate d'ammonium.

Les rapports des espèces dans le milieu réactionnel sont :

$$C_2O_4^=/Y = 2,54$$

$$NH_4^+/Y = 3,26$$

L'oxyde d'yttrium obtenu par calcination du précipité récupéré selon le procédé décrit dans les exemples précédents présente un diamètre moyen des particules égal à 3,5 µm et un $\frac{\sigma}{m} = 0,56$

## Revendications

1. Procédé de fabrication d'un oxalate double de terre rare et d'ammonium caractérisé en ce qu'il consiste :

   - à mélanger, en milieu aqueux, au moins un composé capable de libérer des ions oxalates en solution, ce composé étant autre que l'oxalate d'ammonium, avec au moins un composé de terres rares soluble dans l'eau et un sel d'ammonium, ce sel étant autre que l'oxalate d'ammonium;
   - à séparer le précipité obtenu, les concentrations en ions terres rares (TR) et ammonium étant choisies de telle manière que les rapports molaires $C_2O_4^=$ /TR et $NH_4^+$/TR à la fin de la précipitation, soient supérieurs ou égaux à 2;
   - et, éventuellement sécher ledit précipité.

2. Procédé selon la revendication 1 caractérisé en ce que le sel d'ammonium est choisi parmi le nitrate d'ammonium, le chlorure d'ammonium, l'acétate d'ammonium.

3. Procédé selon la revendication 1 ou 2 caractérisé en ce que le sel d'ammonium et le composé de terres rares sont mélangés en milieu aqueux pour former une première solution .

4. Procédé selon la revendication 3 caractérisé en ce que le composé capable de libérer en solution des ions oxalates est ajouté à la première solution .

5. Procédé selon la revendication 3 caractérisé en ce que le composé capable de libérer des ions oxalates est choisi parmi l'acide oxalique cristallisé ou en solution, les oxalate d'alcalins.

6. Procédé selon l'une des revendications 1 à 3 caractérisé en ce que le composé capable de libérer des ions oxalates est dissous dans l'eau pour former une seconde solution, la première solution étant ajoutée dans ladite seconde solution .

7. Procédé selon l'une des revendications précédentes, caractérisé en ce que le sel soluble de terres rares est choisi dans le groupe des nitrates, chlorures, sulfates ou un mélange de sels de terres rares.

8. Procédé selon la revendication 7 caractérisé en ce que les composés de terres rares sont le nitrate d'yttrium, nitrate d'europium, nitrate de lanthane, nitrate de néodyme, nitrate de dysprosium, cérium, gadolinium, terbium ou un mélange de ceux-ci.

9. Procédé selon l'une des revendications précédentes caractérisé en ce que la précipitation est réalisée à une température comprise entre 30°C et 90°C, de préférence entre 35°C et 70°C .

10. Procédé selon l'une des revendications précédentes caractérisé en ce que la séparation du précipité est réalisée entre 5 min et 2 H après la fin de précipitation .

11. Procédé de fabrication d' oxyde de terres rares, caractérisé en ce qu'il consiste à calciner l'oxalate double d'ammonium et de terre rare obtenu selon l'une des revendications précédentes, après un séchage éventuel.

12. Procédé selon la revendication 11 caractérisé en ce que la température de calcination est comprise entre 600 et 1200°C, de préférence entre 800°C et 1000°C.

## Claims

1. A process for the production of a double oxalate of a rare earth and ammonium, characterized in that it consists of:

   - mixing, in an aqueous medium, at least one compound which can liberate oxalate ions in solution, the compound being other than ammonium oxalate, with at least one rare earth compound which is soluble in water and with an ammonium salt, the salt being other than ammonium oxalate;
   - separating the precipitate obtained, the concentrations of the rare earth ions (RE) and ammonium ions being selected such that the $C_2O_4^=$/RE and $NH_4^+$/RE molar ratios when precipitation is complete are greater than or equal to 2; and
   - optionally, drying said precipitate.

2. A process according to claim 1, characterized in that the ammonium salt is selected from ammonium nitrate, ammonium chloride and ammonium acetate.

3. A process according to claim 1 or claim 2, characterized in that the ammonium salt and the rare earth compound are mixed in an aqueous medium to form a first solution.

4. A process according to claim 3, characterized in that the compound which can liberate oxalate ions in solution is added to the first solution.

5. A process according to claim 3, characterized in that the compound which can liberate oxalate ions is selected from oxalic acid, crystallised or in solution, and alkaline oxalates.

6. A process according to any one of claims 1 to 3, characterized in that the compound which can liberate oxalate ions is dissolved in water to form a second solution, the first solution being added to said second solution.

7. A process according to any one of the preceding claims, characterized in that the soluble rare earth salt is selected from the group formed by rare earth nitrates, chlorides, or sulphates or a mixture of rare earth salts.

8. A process according to claim 7, characterized in that the rare earth compounds are yttrium nitrate, europium nitrate, lanthanum nitrate, neodymium nitrate, dysprosium nitrate, cerium nitrate, gadolinium nitrate, terbium nitrate, or a mixture thereof.

9. A process according to any one of the preceding claims, characterized in that precipitation is carried out at a temperature which is in the range 30°C to 90°C, preferably in the range 35°C to 70°C.

10. A process according to any one of the preceding claims, characterized in that the precipitate is separated 5 minutes to 2 hours after precipitation is complete.

11. A process for the production of rare earth oxides, characterized in that it consists of calcining the double oxalate of ammonium and a rare earth obtained according to any one of the preceding claims, after optional drying.

12. A process according to claim 11, characterized in that the calcining temperature is in the range 600°C to 1200°C, preferably in the range 800°C to 1000°C.

**Patentansprüche**

1. Verfahren zur Herstellung eines Doppeloxalates von seltenen Erden und Ammonium, dadurch gekennzeichnet, daß es besteht in:

   - dem Vermischen im wäßrigen Medium von mindestens einer Verbindung, die fähig ist, Oxalationen in Lösung freizusetzen, wobei diese Verbindung anders ist, als das Ammoniumoxalat, mit mindestens einer Verbindung der seltenen Erden, die in Wasser und in einem Ammoniumsalz löslich ist, wobei dieses Salz anders ist, als das Ammoniumoxalat,
   - dem Abtrennen des erhaltenen Niederschlages, wobei die Konzentrationen an Ionen der seltenen Erden (TR) und Ammonium in der Weise gewählt werden, daß die molaren Verhältnisse $C_2O_4^-$/TR und $NH_4^-$/TR am Ende der Fällung höher oder gleich 2 betragen;
   - und gegebenenfalls dem Trocknen des genannten Niederschlages.

2. Verfahren nach Anspruch 1, dadurch gekennzeichnet, daß das Ammoniumsalz unter Ammoniumnitrat, Ammoniumchlorid und Ammoniumacetat ausgewählt wird.

3. Verfahren nach Anspruch 1 oder 2, dadurch gekennzeichnet, daß das Ammoniumsalz und die Verbindung der seltenen Erden im wäßrigen Medium vermischt werden, um eine erste Lösung zu bilden.

4. Verfahren nach Anspruch 3, dadurch gekennzeichnet, daß die Verbindung, die fähig ist, in Lösung Oxalationen freizusetzen, zu der ersten Lösung gegeben wird.

5. Verfahren nach Anspruch 3, dadurch gekennzeichnet, daß die Verbindung, die fähig ist, Oxalationen freizusetzen, unter Oxalsäure, kristallin oder in Lösung, und den Alkalioxalaten ausgewählt wird.

6. Verfahren nach einem der Ansprüche 1 bis 3, dadurch gekennzeichnet, daß die Verbindung, die fähig ist, Oxalationen freizusetzen, in Wasser aufgelöst wird, um eine zweite Lösung zu bilden, wobei dann die erste Lösung in die genannte zweite Lösung gegeben wird.

7. Verfahren nach einem der vorstehenden Ansprüche, dadurch gekennzeichnet, daß das lösliche Salz der seltenen Erden aus der Gruppe der Nitrate, Chloride, Sulfate oder einer Mischung der Salze der seltenen Erden gewählt wird.

8. Verfahren nach Anspruch 7, dadurch gekennzeichnet, daß die Verbindungen der seltenen Erden Yttriumnitrat, Europiumnitrat, Lanthannitrat, Neodymnitrat, Nitrat von Dysprosium, Cer, Gadolinium, Terbium oder eine Mischung von denen sind.

9. Verfahren nach einem der vorstehenden Ansprüche, dadurch gekennzeichnet, daß die Fällung bei einer Temperatur zwischen 30 °C und 90 °C, vorzugsweise zwischen 35 °C und 70 °C realisiert wird.

10. Verfahren nach einem der vorstehenden Ansprüche, dadurch gekennzeichnet, daß die Abtrennung des Niederschlages zwischen 5 Minuten und 2 Stunden nach dem Ende der Fällung realisiert wird.

11. Verfahren zur Herstellung von Oxid der seltenen Erden, dadurch gekennzeichnet, daß es darin besteht, das nach einem der vorstehenden Ansprüche erhaltene Doppeloxalat von Ammonium und seltenen Erden gegebenenfalls nach einer Trocknung zu kalzinieren.

12. Verfahren nach Anspruch 11, dadurch gekennzeichnet, daß die Temperatur der Kalzinierung zwischen 600 °C und 1200 °C, vorzugsweise zwischen 800 °C und 1000 °C liegt.

Fig. 1